# EUROPEAN PATENT APPLICATION

(11) **EP 3 154 007 A1**
(43) Date of publication of application: **12.04.2017**
(21) Application number: 15188992.0
(22) Date of filing: 08.10.2015
(51) Int. Cl.: G06Q 10/08, G06Q 50/22

(54) **METHOD, MODULE AND SYSTEM FOR MANAGING LOGISTICS AND DISTRIBUTION SERVICES OF PRESCRIBED PHARMACEUTICALS BETWEEN A HEALTH CARE PROVIDER, A PHARMACY AND A PATIENT**

(71) Applicant: Frisq AB, 111 20 Stockholm (SE)
(72) Inventor: CAESAR, Jörgen, 755 98 UPPSALA (SE); JANSSON, Tomas, 167 63 BROMMA (SE)
(74) Representative: Zacco Sweden AB

(57) **Abstract**

The present invention relates to a method, a module and a system for managing logistics and distribution services of prescribed pharmaceuticals between a health care provider, a pharmacy and a patient. The health care provider and the pharmacy are in connection with a health record service which is adapted to receive information of medical prescriptions issued to the patient from the health care provider. The health care provider has a module installed in connection with its patient record, so as to enable a comparison of patients for which at least one new medical prescription has been issued from patients for which no such prescription has been issued. This comparison is then used to notify the relevant group of patients and thus increases the level of service and accessibility while maintaining the personal integrity.

## Description

### TECHNICAL FIELD

The present invention relates to a method, a module and a system for managing logistics and distribution services of prescribed pharmaceuticals between a health care provider, a pharmacy and a patient.

### BACKGROUND ART

Parallel importation of pharmaceuticals and its compliance with acknowledged international trade principles, regional and national legislation has been a matter of debate and regulations at least for a decade by now. Several methods and systems have been proposed for managing distribution and pricing in relation to pharmaceuticals.

One of the proposed systems is described in the published US patent application 2006/0259363. The application discloses a system for controlling the distribution of pharmaceuticals that involves tracking the distribution of pharmaceutical products and managing pharmaceutical rebates via the internet. The system provides for the verification, payment and auditing of the rebates. Before the pharmacy can participate in the rebate scheme they must first undergo an initial registration process. Following registration, the pharmacy may purchase pharmaceutical products which are supplied from the manufacturer via a supply chain. The pharmaceuticals are supplied at a standard supply price, irrespective of the country in which the retailers are located. When the pharmaceuticals are supplied to an end user, e.g. a patient, the pharmacy enters a rebate claim, which is processed by an international clearing centre. Provided the processing has a successful outcome, the pharmacy receives a rebate payment, the size of which depends upon the country in which the pharmacist is located. This allows the dispensing of prescription pharmaceuticals at a discount to the patient and reduces the prevalence of parallel importation.

US patent 8,433,587 describes a related method for communicating a medical prescription with a mobile processing system. According to the method described, a first request to fulfil the medical prescription is received from the mobile processing system. This first request is formatted in an instant messaging protocol and includes a mobile processing system identifier and a transaction identifier. A patient identifier that is associated with the mobile processing system identifier is identified, and transactions associated with the patient identifier are accessed to identify a transaction that is associated with the transaction identifier. The transaction then is transmitted in a second request to a pharmacy system that is configured to fulfil the medical prescription.

None of the mentioned records of prior art allows for an on-line efficient ordering and distribution service which is adapted to protect the identity, medical and health-related information, and thus the personal integrity of the patient. It is therefore and object of the invention to provide relevant methods and means in order to alleviate the previously mentioned shortcomings associated with prior art technology, in particular with respect to maintaining the personal integrity.

### SUMMARY OF THE INVENTION

In view of the above, not only human values constitute the basis for the invention. Also legislative and regulatory reasons have been considered carefully, since any method and or system must protect the personal integrity to comply with national and international legislation. Needless to say, this be may an absolute necessity for approval by FDA and related organisations to allow a product or service to be introduced on the market.

The present invention relates to a method, a module and a system for managing logistics and distribution services of prescribed pharmaceuticals between a health care provider, a pharmacy and a patient, the health care provider and the pharmacy being in connection with a health record service which is adapted to receive information of medical prescriptions issued to the patient from the health care provider, the health care provider having a module installed in connection with its patient record, the method comprising the steps of:
an interim node transferring information about the patient, his/her health condition and/or medical prescriptions to a central server arrangement in connection with the interim node, and authorising the central server arrangement to forward information about the identity of the patient,
the module installed in connection with the patient record of the health care provider being adapted for:
   creating a first list of identities of patients contained in the patient record of the health care provider, the list including identities of patients who have received at least one new medical prescription,
   truncating the identities of the patients contained in the first list using an arbitrary truncation algorithm so as to maintain anonymity of the patients, thereby creating a second list,
   sending the second list including truncated identities of patients to the central server arrangement in which a match is made between the second list and a third list, the third list containing all patients for which information has been transferred from the interim node to the central server arrangement, whereby each match creates an entry in a forth list of matching identities between the second and third lists,
   if matching identities between the fourth and first lists exist, the fourth list is transferred to the central server arrangement, which extracts patients, for which the authorisation has been made, thereby creating a fifth list exclusively containing identities of authorised patients for which at least one new medical prescription has been made,
the central server arrangement notifying the interim node of patients from the fifth list, and
the patient entering into the interim node and retrieving information from the health record service via the central server arrangement, about his/her at least one new medical prescription.

In accordance with a related aspect of the invention, a patient initially empowers the interim node to perform logistics and distribution services on his/her behalf. An advantage of this empowering feature is that personal data, such as a patient's identity and health related data, relating to patients who have not given their consent to being processed is never processed, neither by the interim node nor by any other means. This is a substantive advantage of the present invention because refraining from processing data of non-empowering patients enhances compatibility with general legislation such as national and international Personal Data Acts and the Patient Data Acts and the likes. One of the main principles of protective data acts is to prevent unauthorised interlinking of sensitive data, which could otherwise be heavily abused. The present invention allows a patient to, explicitly and in advance, decide and authorise the enabling service according to the invention. The patient makes an own decision based on whether he or she considers the benefits of the service outweigh any perceived insecurity relating to management of personal health-related information, which is sensitive to many people.

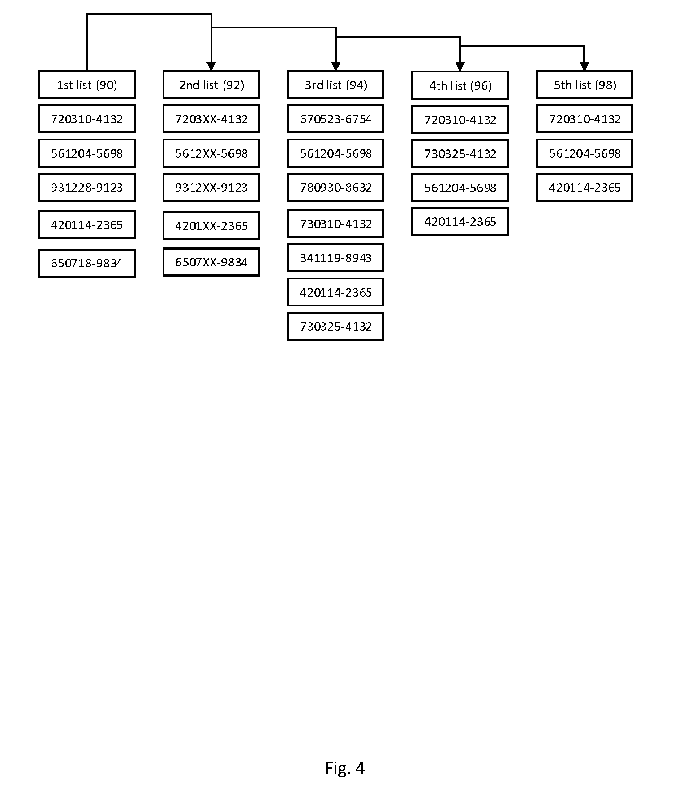

The truncation can be made using any means or by any anonymising algorithm available. One a simple and exemplifying method could be to hide at least one character or digit contained in a personal identification number. With particular reference to figure 4, this is illustrated by exchanging 2 out of 10 digits with an X.

The present invention is intended to provide means and methods to enhance distribution efficiency and also enable an increased service level as perceived by the patient. While doing that, it also provides a completely non-intrusive service and maintains the anonymity of patients. Yet another advantage brought to the patients and medical prescription systems and methods is that the invention helps alleviating the problem of how to avoid the risk of multiple prescriptions in parallel.

In accordance with a related embodiment of the invention, the invention further comprises the steps of:
the interim node providing the patient with at least one option of pharmacy for ordering and having pharmaceuticals delivered for which a new medical prescription exists,
the patient executing the choice of pharmacy for ordering and delivery of pharmaceuticals,
creating and transmitting an order to the pharmacy of choice, and
the pharmacy of choice expediting and delivering the ordered pharmaceuticals to the patient followed by notifying the health record service that the new prescription has been expedited so as to avoid ordering in excess of an intended medical prescription. The patient may, as a further option, be provided with a number of alternative options of pharmacies to order from.

This embodiment assists the patient in suggesting or choosing the most convenient pharmacy to order from, which choice can be based on a multitude of criteria, such as location of the pharmacy, price level, service level or the present assortment of pharmaceuticals.

In accordance with yet another embodiment, the interim node is configured as an application (app) on a smart phone. This is beneficial to the both patient and the service provider, because the patient has easy access to services from any location and the service provider has a well-defined platform for which at least one application can be developed.

According to one embodiment of the invention, the service provider has adapted the application and surrounding systems to a health record service which in some areas and jurisdictions is a mandatory public record service, which collects information from health care providers about prescriptions of patients within a predefined group. The predefined group may furthermore be determined based on at least one of geography, demography, medical history, medical insurance etc.

Feasible is that the health care provider is a medical service provider, such as a medical doctor, nurse, clinic etcetera, but the health care provider may also be a veterinary service generally, and the patient may be the owner of a pet or a farmer, then typically in possession of a herd of animals.

### BRIEF DESCRIPTION OF THE DRAWINGS

Figure 1 is a schematic diagram illustrating the means according to the present invention and their interconnection.
Figure 2 is a signalling scheme sequentially illustrating the exchange of information between the comprised transmission means according to the present invention.
Figure 3 is a flow chart schematically illustrating the method for managing logistics and distribution services of prescribed pharmaceuticals according to the present invention.
Figure 4 illustrates the first, second, third, fourth and fifth lists with exemplifying anonymization and exemplifying personal identification numbers according to one embodiment of the present invention.

### DETAILED DESCRIPTION

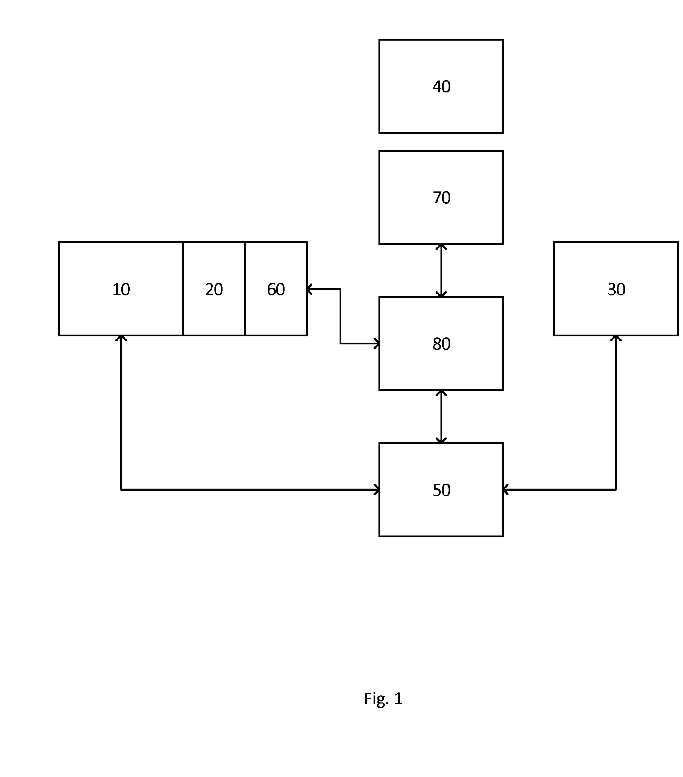

The present invention relates to a method, a module and a system for managing logistics and distribution services of prescribed pharmaceuticals. Referring to figure 1 in particular, the involved in the managing process are a health care provider 10, a pharmacy 30, in particular an expediting system of the pharmacy, and a patient 40. The health care provider and the pharmacy are both adapted to enable an exchange of information with a health record service 50, which is a public mandatory service in some but not all countries. The health record service is provided to receive information of medical prescriptions issued to a patient from the health care provider. In accordance with the present invention, the health care provider has agreed to installation of a specialised software module 60 (logics module) in connection with its patient record 20 so as to provide the desired functionality according to the present invention. This installation is typically made by downloading a computer program which is then installed locally in connection with the patient record.

Further with reference to figure 1, the patient has access to an interim node, preferably an application installed on a smartphone or other similar handheld device. However, a web based service is just as conceivable depending on the smartphone density in a certain area or the demographic group of potential customers, for example elderly who are typically not in possession of smartphones to the extent of younger potential customers. If not accessible via a smartphone, the interim node may be connected in other ways as long as it is able to exchange information bi-directionally with the eco system around smartphones and similar portable or handheld devices. This interim node is adapted to exchange information with a central server arrangement 80, preferably a storage server connected to the Internet.

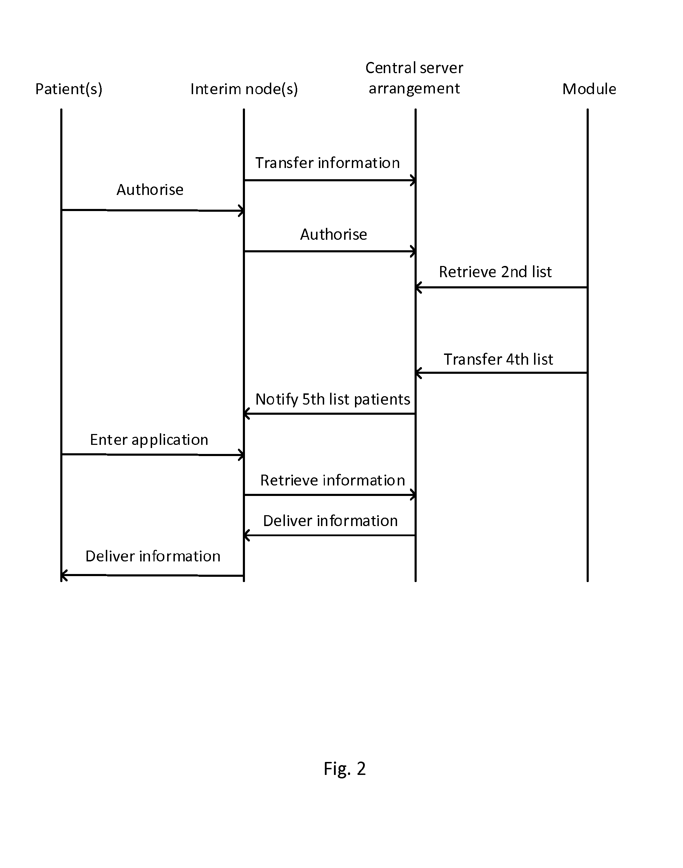

Referring to figure 2 in particular, initially a patient is asked to give his/her consent to an interim node. The consent is forwarded to the central server arrangement, and is an absolute prerequisite for the interim node to share information concerning the patient, his/her health condition and/or medical prescriptions to the central server arrangement 80, from which information can be redistributed, provided prior authorisation is given by the patient.

Once the specialised software module has been installed in connection with the patient record of the healthcare provider, a first list 90 is created of identities of patients contained in the patient record of the health care provider. This first list includes identities of patients who have received at least one new medical prescription.

A second list 92 is created by truncating the identities of the patients so as to maintain their anonymity using a standard algorithm for anonymizing personal identification numbers or similar personal identity markers.

In a next sequential step, a third list 94 is retrieved from the from the central server arrangement, the list including identities of patients, who have authorised transfer of their health-related information and for which information has consequently been transferred from the interim node to the central server arrangement.

It is determining whether any of truncated identities of patients of the first list matches with identities of patients of the second list by means of a simple comparison process. In case the comparison of identities of the first and second lists results in a match, a fourth list 96 is created, containing the matching identities between the first and the second lists.

Resulting from the anonymization carried out when creating the first list, the third list may still contain also identities of patients who have not given their consent to their personal health-related information being transferred. In order to avoid this from occurring, the central server arrangement filters out the patients who have authorised forwarding of their personal data from the third list and creates a fifth list 98.

This fifth list now contains an extraction of patients who have given their consent and who have gotten at least one new medical prescription. The central server arrangement uses the fifth list to notify interim node recipients, i.e. preferably and most likely a smartphone application. Each interim node is associated with a particular patient who has expressly in advance given his/her consent to having their personal data transferred and thus to taking part in the service provided. As soon as a patient enters into the interim node, information is retrieved from the health record service via the central server arrangement, about the at least one new medical prescription.

By means of the present invention, reservation and distribution of pharmaceuticals to patients can be provided in a more efficient and secure manner, by means of a web portal or a smartphone bases application. Distribution can be made to the patient's home of to another location at the choice off the patient's. A web portal and the smartphone application are aimed primarily at small pharmacy operators who cannot or lacks interest in building its own complete e-commerce web site, which for obvious reasons may be out of reach for such an entity.

The present invention is adapted to manage both human and animal prescriptions and/or prescription orders. It may also comprise logic for subscription of so-called iterated prescriptions.

The operation of any e-commerce or electronic payment systems, short message services or other text messaging service components of mobile phones, smartphones, web-based services and mobile communication systems etcetera, will not be described here in more detail.

The specialised software module (logics module) is adapted to, at specified intervals poll the patient record of the health care provider to determine whether any new prescriptions have been issued with respect to any patients who have given their consent to being processed by the service. In the event that at least one new prescription has been issued, information is provided about the user's personal identity and issued prescription to the central server arrangement.

The logistics and distribution management service according to the present invention operates as follows:
Via an order portal in a browser or a smartphone application, a patient or his/her delegate/agent, may enter medical prescriptions in a public or official medical prescription record and for each one of the prescribed pharmaceuticals order home delivery or pre-order the delivery of a pharmacy at the choice of the patient. This is of course applicable both to both human and animal prescriptions. Any person or authority may be empowered to accomplish the same services for example elderly, such as parents in need of assistance or medical case or children.

The method and system according to the invention is adapted to calculate an appropriate time of delivery and the patient can place an order with a particular timing in accordance with the anticipated future need. This allows the patient to order delivery of scheduled future medical prescriptions. Home delivery may furthermore be pre-initiated during the occasion when care is given.

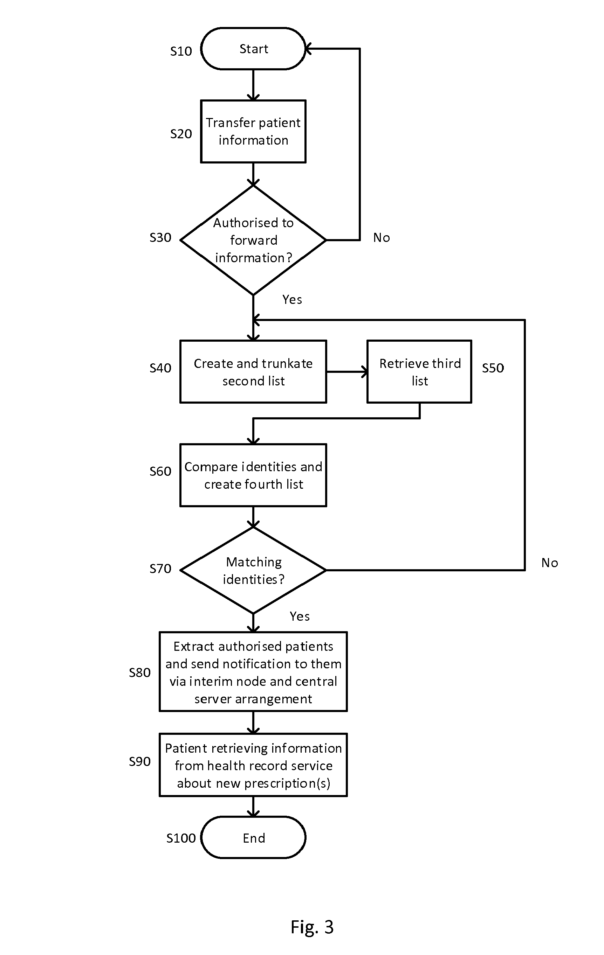

With particular reference to figure 3, the sequential method according to the invention is as follows:
In a first step, the sequence starts S10 with the patient enrolling with the service provided and by downloading and installing the interim node on his/her computer of mobile device, such as typically installing an application on a smartphone. After that, the patient enters his/her credentials including any health-related information and is also explicitly asked to authorise personal and health-related information to be forwarded so as so enable relevant services to operate. Then, the interim node shares information about the patient and his/her health-related data with the central server arrangement. Provided authorisation has been given S30, the central server arrangement forwards information S20 to at least one specialised software module associated with the patient record of one or plural health care providers. In case no such authorisation is given, the patient does not consent to forwarding information in the system and thus no distribution is made of personal information.

Provided the patient has authorised distribution of his/her personal and health-related information, the specialised software module, i.e. the embedded logics module, creates a first list S40 of patients who have received at least one new medical prescription. This list is modified in a second list containing only unidentifiable items due to a truncation of data strings or other method of anonymising personal data. Thus, no personal, medical or health-related information is revealed. In a next step, the module retrieves a third list S50 from the central server arrangement, the list containing personal, medical and or health-related information relating to patients who have given their explicit consent by authorising transmission of such information.

The module then compares data between the second truncated list and the third list S60 so as to determine whether there are any matching identities. A fourth list is created containing the matching identities. If any such identities exist S70, this indicates that at least one of the enrolled patients have received at least one new medical prescription. However, due to the truncation, another extraction needs to be made, which is a comparison of the fourth list of identities with the complete data strings contained in the module before truncation, which is made and finally results in a fifth list. This final list is used to determine correct recipients and results in a notification to be sent S80 to the affected patient from the module via the central server arrangement and the interim node. As soon as the patient enters into the application on his/her smartphone, or alternatively any other arrangement such as a web application on a PC, information about the new prescription is retrieved S90 and the patient is informed about the new prescription and is allowed to act accordingly in response to that.

By that, the sequence ends S100 unless supplemented with a method for a pharmacy to utilise when expediting and managing payment and delivery if the prescribed pharmaceuticals or related items.

One possible action could be to order and settle payment by means of state of the art e-commerce systems available on the market. The prescribed pharmaceuticals will then be delivered either to the patient personally when visiting the pharmacy of choice, or distributed to his/her home or other location if that is mode convenient.

Access to the previously mentioned web-based service, is preferably realised in the form of a node between the patient and the service provider, which is to accessed in the form of a so-called web portal or application, may be provided via a link from a pharmacy operator website or alternatively via an independent operator. In the first case, the patient sees only the current operator's pharmacies, and in the second case all of the pharmacies in a certain region, county or even state or country may be made available.

A medical prescription via any of the access routes mentioned above, labelled either for home delivery or for pre-ordering, is stored together with information about the medical prescription and the patient in the central server arrangement, together with information about which pharmacy is chosen to expedite this order. This information is hereafter referred to as orders. Also name and address information along with prescription identity (recipe identity for animals) is stored, with the aim to provide adequate and relevant information to the patient or recipes to be identified as orders to be expedited by a particular pharmacy of choice.

Each pharmacy, which is affiliated to the provided service, periodically scans the central server arrangement for orders that are possible to expedite from the particular pharmacy. When such prescriptions are found, and have not been reserved by the pharmacy that identifies the prescription in the health care record register, it is to be dispatched it in the regular expedition systems of the pharmacy. This step is followed by expedition and delivery of the relevant pharmaceutical to for instance a pharmacy logistics partner for delivery to the patient's selected address.

If the prescription is instead marked for pre-ordering can this be accomplished in an order according to the pharmacy of choice until the patient, notified by SMS/email/application or similar arrives.

Payment is made either through the own partners of the pharmacy for the billing/payment or through a proprietary service for billing/card payments via a payment provider, chosen by default of the proprietary system or by the patient.

Web services are used as communication protocols, both internally and from the central server arrangement, when communicating with pharmacy services.

## Claims

1. A method for managing logistics and distribution services of prescribed pharmaceuticals between a health care provider (10), a pharmacy (30) and a patient (40), the health care provider and the pharmacy being in connection with a health record service (50) which is adapted to receive information of medical prescriptions issued to the patient from the health care provider, the health care provider having a module (60) installed in connection with its patient record (20), the method comprising the steps of:
an interim node (70) transferring information about the patient, his/her health condition and/or medical prescriptions to a central server arrangement (80) in connection with the interim node, and authorising the central server arrangement to forward information about the identity of the patient,
the module installed in connection with the patient record of the health care provider being adapted for:
creating a first list (90) of identities of patients contained in the patient record of the health care provider, the list including identities of patients who have received at least one new medical prescription,
truncating the identities of the patients contained in the first list using an arbitrary truncation algorithm so as to maintain anonymity of the patients, thereby creating a second list (92),
sending the second list including truncated identities of patients to the central server arrangement in which a match is made between the second list and a third list (94), the third list containing all patients for which information has been transferred from the interim node to the central server arrangement, whereby each match creates an entry in a forth list (96) of matching identities between the second and third lists,
if matching identities between the fourth and first lists exist, the fourth list is transferred to the central server arrangement, which extracts patients, for which the authorisation has been made, thereby creating a fifth list (98) exclusively containing identities of authorised patients for which at least one new medical prescription has been made,
the central server arrangement notifying the interim node of patients from the fifth list, and
the patient entering into the interim node and retrieving information from the health record service via the central server arrangement, about his/her at least one new medical prescription.

2. Method according to claim 1, wherein the arbitrary truncation algorithm is any standard algorithm applied for truncation or anonymization of personal identity numbers.

3. Method according to claim 1, wherein the patient initially empowers the interim node to perform logistics and distribution services on his/her behalf.

4. Method according to claim 1, further comprising the steps of:
the interim node providing the patient with at least one option of pharmacy for ordering and having pharmaceuticals delivered for which a new medical prescription exists,
the patient executing the choice of pharmacy for ordering and delivery of pharmaceuticals,
creating and transmitting an order to the pharmacy of choice, and
the pharmacy of choice expediting and delivering the ordered pharmaceuticals to the patient followed by notifying the health record service that the new prescription has been expedited so as to avoid ordering in excess of an intended medical prescription.

5. Method according to claim 1, wherein the interim node is configured as an application (app) on a smart phone.

6. Method according to claim 1, wherein the health record service is a mandatory public record service, which collects information from health care providers about prescriptions of patients within a predefined group.

7. Method according to claim 6, wherein the predefined group is determined based on at least one of geography, demography, medical history, medical insurance etc.

8. Method according to claim 1, wherein the patient is provided with a number of alternative options of pharmacies to order from.

9. Method according to claim 1, wherein the health care provider is a veterinary service and the patient is the owner of a pet or a farmer, typically in possession of a herd of animals.

10. Module for managing logistics and distribution of prescribed pharmaceuticals between a health care provider (10), a pharmacy (30) and a patient (40), the module comprising physical means for realising the method according to anyone of claims 1-9.

11. System for managing logistics and distribution of prescribed pharmaceuticals between a health care provider (10), a pharmacy (30) and a patient (40), the system comprising physical means to achieve functionality so as to carry out the method according to anyone of claims 1-9.
